# EUROPÄISCHE PATENTANMELDUNG

(11) **EP 3 628 231 A1**
(43) Veröffentlichungstag der Anmeldung: **01.04.2020**
(21) Anmeldenummer: 18197560.8
(22) Anmeldetag: 28.09.2018
(51) Int. Cl.: A61B 6/04, A61N 5/10

(54) **MEDIZINISCHE MATRATZE UND VERFAHREN ZUR ECHTZEITÜBERWACHUNG**

(71) Anmelder: Siemens Healthcare GmbH, 91052 Erlangen (DE)
(72) Erfinder: Britzen, Stefan, 91054 Buckenhof (DE)

(57) **Zusammenfassung**

Medizinische Matratze zur Bestimmung von Position und/oder Positionsänderung eines auf der Matratze gelagerten Patienten, welche Matratze zumindest teilweise aus röntgendurchlässigen Materialien ausgebildet ist, aufweisend eine Oberseite, welche als Liegefläche für den Patienten ausgebildet ist und eine der Oberseite gegenüber liegende Unterseite, eine Vielzahl von Luftkanälen, welche über zumindest einen Teilabschnitt der Matratze in einer Matrix verteilt derart angeordnet ist, dass die Luftkanäle sich von der Unterseite zur Oberseite der Matratze erstrecken, wobei jeder Luftkanal an seinem ersten Ende im Bereich der Oberseite eine Öffnung aufweisen und an seinem zweiten Ende an einen Luftversorgungsschlauch angeschlossen ist, eine Luftversorgungseinrichtung, welche über die Luftversorgungsschläuche Luft in die Luftkanäle pumpt, Sensoren zur Messung von Strömungs- und/oder Strömungsänderungskenngrößen in den Luftkanälen, und eine zugeordnete Auswertungseinheit zur Auswertung der von den Sensoren gemessenen Strömungskenngrößen und/oder Strömungsänderungskenngrößen hinsichtlich einer Position und/oder Positionsänderung des auf der Matratze gelagerten Patienten.

## Beschreibung

Die Erfindung betrifft eine Medizinische Matratze zur Bestimmung von Position und Positionsänderung eines auf der Matratze gelagerten Patienten gemäß dem Patentanspruch 1 sowie einem Verfahren zur Echtzeitüberwachung einer Positionierung eines Patienten während einer medizinischen Diagnose und/oder Therapie gemäß dem Patentanspruch 13.

Während medizinischer Interventionen an einem Patienten werden zur Navigation von medizinischen Instrumenten im Körper des Patienten (z.B. in Kopf oder Herz) mittels fluoroskopischer (zweidimensionaler) Durchleuchtungsbildgebung Echtzeit-Röntgenbilder aufgenommen. Verglichen mit Bildern aus 3D-Modalitäten (z.B. Magnetresonanzbildgebung, Computertomographie oder 3D-Angiographie) zeigen diese zweidimensionalen Röntgenbilder zwar keine räumlichen (3D) Details, sie sind jedoch schneller verfügbar, hochaufgelöster, minimieren die Strahlenbelastung für Patient und Arzt und bilden interventionelle Devices wie Operationsinstrumente, Katheter, Führungsdrähte usw. in Quasi-Echtzeit ab. Idealerweise wird die räumliche Information dadurch zurückgewonnen, dass prä-operativ aufgenommene Volumenbilder mit den zweidimensionalen Echtzeit-Röntgenbildern registriert und überlagert werden (2D3D-Registrierung).

Die Kombination von co-registrierten 2D und 3D Bildern erlaubt dem Arzt eine sehr gute Orientierung im Patienten. Wünschenswert ist, dass eine solche Registrierung möglichst genau und ohne manuelle Interaktion durchgeführt werden kann. Des Weiteren müssen eventuelle Patientenbewegungen registriert und dementsprechend durch das System oder den User reagiert werden. Im derzeitigen klinischen Setup werden Patientenbewegungen nicht automatisch erkannt. Die 2D3D Registrierung erfolgt rein bildbasiert, welche vom User manuell angestoßen werden muss.

Der User muss im Anschluss z.B. manuell überprüfen, ob die Registrierung weiterhin passt. Nach erkannter Misregistrierung muss er dann manuell die Registrierung korrigieren, was zu Ungenauigkeiten und Verzögerungen im Arbeitsablauf führt. Besonders störend ist dies bei zyklisch wiederkehrenden Patientenbewegungen, wie zum Beispiel der Atmung oder dem Herzschlag. Hierdurch wird eine ständige Misregistrierung und Ungenauigkeit erzeugt, vor allem im Bereich des Thorax oder des Abdomen. Neben den Interventionen leidet auch die Qualität der Strahlentherapie bei Lungen- bzw. Bronchialkarzinomen unter den Atembewegungen. Hier gibt es verschiedene Ansätze zur Kompensation, die allerdings alle nicht zuverlässig zufriedenstellend funktionieren und dadurch möglicherweise auch gesundes Gewebe bei der Strahlentherapie geschädigt werden kann.

Aus dem Stand der Technik sind folgende Lösungen zur Überwachung von Patientenposition und -positionsänderungen bekannt:
(a) Eine Registrierung des Volumens zum C-Arm mit "optischer Oberflächenerkennung" z. B. durch 3D Kameras mit Sicht auf den Patienten; problematisch ist bei diesen Methoden jedoch, dass diese eine freie Sicht auf den Patienten fordern, was gerade in OPs aufgrund der Vielzahl der Gerate kaum möglich ist.
(b) Verschiedene Methoden zur 2D3D- oder 3D3D-Registrierung
(c) Eine Erfassung der Atmung durch Dehnungsdetektoren, die um die Brust gespannt werden und so die Atembewegung detektieren; der Nachteil liegt in der limitierten Art der eindimensionalen Bewegungserfassung, die die eigentliche Komplexität der Atmung nicht adäquat erfasst.
(d) Bei der Strahlentherapie behilft man sich mit invasiv gesetzten Markern (Fiducials) in der Nähe vom oder im Tumor, sowie einer parallelen kV-Bildgebung zur Verfolgung des Ziels. Beide Techniken bergen Gefahren und Limitationen (Pneumothorax beim Setzen der Fiducials, bzw. Nicht-Sichtbarkeit des Tumors unter kV-Bildgebung), die ein erhöhtes Risiko für den Patienten bedeuten.
(e) Terahertzkameras oder ähnliche Sensoren (z.B. im Einsatz der Flughafensicherheit).

Es ist Aufgabe der vorliegenden Erfindung, eine alternative Vorrichtung bereitzustellen, welche eine einfache und zuverlässige Überwachung von Positionen und Positionsänderungen eines Patienten während eines Eingriffs mit fluoroskopischer Bildgebung ermöglicht; des Weiteren ist es Aufgabe der Erfindung, ein Verfahren zur Echtzeitüberwachung eines Patienten während einer medizinischen Diagnose und/oder Therapie bereitzustellen.

Die Aufgabe wird erfindungsgemäß gelöst durch eine Medizinische Matratze zur Bestimmung von Position und Positionsänderung eines auf der Matratze gelagerten Patienten gemäß dem Patentanspruch 1 und von einem Verfahren zur Echtzeitüberwachung einer Positionierung eines Patienten während einer medizinischen Diagnose und/oder Therapie gemäß dem Patentanspruch 13. Vorteilhafte Ausgestaltungen der Erfindung sind jeweils Gegenstand der zugehörigen Unteransprüche.

Die erfindungsgemäße medizinische Matratze zur Bestimmung von Position und/oder Positionsänderung eines auf der Matratze gelagerten Patienten, welche Matratze zumindest teilweise aus röntgendurchlässigen Materialien ausgebildet ist, weist eine Oberseite, welche als Liegefläche für den Patienten ausgebildet ist und eine der Oberseite gegenüber liegende Unterseite, eine Vielzahl von Luftkanälen, welche über zumindest einen Teilabschnitt der Matratze in einer Matrix verteilt derart angeordnet ist, dass die Luftkanäle sich von der Unterseite zur Oberseite der Matratze erstrecken, wobei jeder Luftkanal an seinem ersten Ende im Bereich der Oberseite eine Öffnung aufweisen und an seinem zweiten Ende an einen Luftversorgungsschlauch angeschlossen ist, eine Luftversorgungseinrichtung, welche über die Luftversorgungsschläuche Luft in die Luftkanäle pumpt, Sensoren zur Messung von Strömungs- und/oder Strömungsänderungskenngrößen in den Luftkanälen, und eine zugeordnete Auswertungseinheit zur Auswertung der von den Sensoren gemessenen Strömungskenngrößen und/oder Strömungsänderungskenngrößen hinsichtlich einer Position und/oder Positionsänderung des auf der Matratze gelagerten Patienten auf.

Mittels der erfindungsgemäßen medizinischen Matratze kann auf einfache Weise während eines medizinischen Diagnose und/oder Therapieverfahrens mit einem Bildgebungsgerät eine Überwachung eines Patienten hinsichtlich einer Positionsänderung bzw. Bewegung durchgeführt werden, so dass weitere Schritte z.B. hinsichtlich einer Warnung oder eines Hinweises oder einer Re-Registrierung durchgeführt werden können. Die Matratze kann dabei Informationen für bzw. über Positionsprofile und Positionsänderungen schnell und in Echtzeit liefern. Eine solche Matratze hat dabei den Vorteil, dass sie im Untersuchungsbereich komplett aus röntgentransparenten Materialien aufgebaut sein kann und somit auch keinerlei Einschränkungen hinsichtlich der Bildgebung zu erwarten sind. Die Matrix von Luftkanälen ist z.B. hinsichtlich ihrer Granularität an die benötigte Messgenauigkeit angepasst aufgebaut. Die Auswerteeinheit kann auch außerhalb der Matratze angeordnet sein und mit dieser lediglich in Kommunikationsverbindung stehen. Unter "Luft" wird hier ein Gas verstanden, welches in größerer Menge eingeatmet werden kann, ohne schädlich für einen Patienten zu sein. Unter Strömungskenngrößen und/oder Strömungsänderungskenngrößen werden Messwerte verstanden, aus welchen Strömungen oder Strömungsänderungen bestimmt werden können, also sowohl direkte Strömungs- und Strömungsänderungsmesswerte also auch andere Messwerte, aus denen Strömungen oder Strömungsänderungen berechnet werden können. So können zum Beispiel auch Drücke oder Druckänderungen gemessen werden.

Nach einer Ausgestaltung der Erfindung werden die Sensoren von Strömungssensoren gebildet. Derartige Sensoren können Luftströmungen und Druckänderungen der Matratze erfassen und liefern damit die Informationen, die notwendig sind, um ein exaktes Positionsprofil zu erstellen bzw. Positionsänderungen des auf der Matratze gelagerten Patienten zu beschreiben. Es können auch Drucksensoren verwendet werden.

Nach einer weiteren Ausgestaltung der Erfindung ist die Auswertungseinheit zur Erstellung eines Positionsprofils des Patienten ausgebildet, insbesondere unter Verwendung von Machine Learning Methoden. Hierfür kann z.B. ein Algorithmus bzw. eine Software verwendet werden, um aus den Strömungskenngrößen ein solches Positionsprofil zu berechnen. Im Rahmen eines Machine Learning oder Deep Learning Verfahrens können bereits mit einer Vielzahl von Datensätzen trainierte Algorithmen verwendet werden.

Nach einer weiteren Ausgestaltung der Erfindung ist die Auswertungseinheit zur Echtzeit-Überwachung von Positionsänderungen des Patienten ausgebildet, insbesondere unter Verwendung von Machine Learning Methoden. Hier werden von den Sensoren registrierte Strömungsänderungskenngrößen ebenfalls von einem Algorithmus bzw. einer Software zu einer Information über eine Positionsänderung bzw. Bewegung des Patienten verarbeitet. Die gesamte Verarbeitungskette kann so schnell erfolgen, dass auch kleinste Bewegungen bzw. kurze und regelmäßige Bewegungen registriert werden. Im Rahmen eines Machine Learning oder Deep Learning Verfahrens können bereits mit einer Vielzahl von Datensätzen trainierte Algorithmen verwendet werden.

Zweckmäßigerweise kann die Matratze zusätzlich eine Speichereinheit zur Speicherung von Daten und Messwerten aufweisen, um die von den Sensoren aufgenommenen Kenngrößen bzw. die ausgewerteten Positionsprofile und Positionsänderungen zu speichern. Die Informationen können auch an weitere Geräte wie z.B. ein Bildgebungsgerät weitergegeben werden.

Nach einer weiteren Ausgestaltung der Erfindung wird die Luftversorgungseinrichtung von einer Pumpe oder einem Kompressor gebildet.

Nach einer weiteren Ausgestaltung der Erfindung sind die Sensoren zur Messung von Strömungs- und/oder Strömungsänderungskenngrößen an oder in den Luftversorgungsschläuchen angeordnet. Auf diese Weise gelingt es auf einfache Weise, nichtröntgentransparente Teile, welche die Sensoren üblicherweise enthalten, außerhalb eines möglichen Röntgenstrahl-Durchleuchtungsbereichs anzuordnen und gleichzeitig aber die Strömungsverhältnisse exakt zu messen. In diesem Zusammenhang ist es vorteilhaft, die Luftversorgungsschläuche außerhalb des Röntgenstrahl-Durchleuchtungsbereichs, insbesondere seitlich(?) der Matratze, zusammenführbar anzuordnen. So können die Luftversorgungsschläuche nicht nur aus dem Durchleuchtungsbereich gehalten werden sondern auch platzsparend und stolpersicher gebündelt werden.

Nach einer weiteren Ausgestaltung der Erfindung erstreckt sich die Matrix von Luftkanälen über zumindest 50% der Matratze. Auf diese Weise können zuverlässig auch kleine Positionsänderungen und Bewegungen des Patienten registriert werden.

Nach einer weiteren Ausgestaltung der Erfindung ist die Matratze zwischen den Luftkanälen aus einem luftdurchlässigen Material, insbesondere einem Schaum, gebildet. Auf diese Weise ist die Matratze einfach und zuverlässig komprimierbar, so dass durch einen entsprechend darauf einwirkenden Druck (Patientengewicht) die Matratze komprimiert wird und in den Luftkanälen ein Druckabfall entsteht, welcher Strömungsänderungen erzeugt. Insbesondere sind auch die Wandungen der Luftkanäle röntgentransparent und komprimierbar ausgebildet. Schaummaterialien sind dabei für einen solchen Einsatz geeignet und für die Anwendung in Matratzen bewährt.

Eine derartige Matratze kann zusätzlich auch durch eine temperaturgeregelte Belüftung für ein Wärmen des Patienten verwendet werden. Aus den Strömungs- und/oder Strömungsänderungskenngrößen kann unter Verwendung einer Kalibrierung auch das Patientengewicht und/oder die Patientengröße gewonnen werden.

Das erfindungsgemäße Verfahren zur Echtzeitüberwachung einer Positionierung eines Patienten während einer medizinischen Diagnose und/oder Therapie mit einem medizinischen Bildgebungsgerät unter Verwendung einer erfindungsgemäßen medizinischen Matratze, auf welcher der Patient gelagert ist, weist die folgenden Schritten auf: Einpumpen von Luft in die Luftkanäle durch die Luftversorgungseinrichtung über die Luftversorgungsschläuche, Messung von Strömungs- und/oder Strömungsänderungskenngrößen in den Luftkanälen, und Auswertung der von den Sensoren gemessenen Strömungs- und/oder Strömungsänderungskenngrößen hinsichtlich einer Position und/oder Positionsänderung des Patienten, wobei die Schritte bis zu einem Abbruchkriterium kontinuierlich und/oder in vorgegebenen zeitlichen Abständen durchgeführt werden. Das Einpumpen wird dabei bevorzugt kontinuierlich durchgeführt, während die Messung und Auswertung bei Bedarf kontinuierlich oder in vorgegebenen zeitlichen Abständen durchgeführt wird.

Nach einer Ausgestaltung der Erfindung wird aus den gemessenen Strömungs- und/oder Strömungsänderungskenngrößen ein Positionsprofil erstellt und für eine Registrierung des medizinischen Bildgebungsgeräts mit der Patientenposition verwendet. Auf diese Weise kann eine exakte Registrierung des Bildgebungsgeräts mit zuvor aufgenommenen Volumenbildern durchgeführt werden.

Nach einer weiteren Ausgestaltung der Erfindung wird ein Hinweis oder eine Warnung weitergegeben, wenn die Auswertung eine Positionsänderung des Patienten ergibt. Hier kann entweder ein Warnsignal an einen User ausgegeben werden oder eine automatische Triggerung einer an die Situation angepassten Aktion durchgeführt werden. So kann in vorteilhafter Weise eine Re-Registrierung des medizinischen Bildgebungsgeräts mit der Patientenposition angetriggert werden, wenn die Auswertung eine Positionsänderung des Patienten ergibt. Hierdurch wird eine kontinuierliche Echtzeitüberwachung von Patientenbewegungen mit automatisch erfolgender Registrierungskorrektur durchgeführt, so dass eine optimale Bildgebung gewährleistet ist. Während medizinischer Interventionen kann sich das medizinische Personal vollständig auf den Eingriff konzentrieren, was die Fehlerquote verringert und eine verbesserte Versorgung des Patienten fördert.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Ansicht einer erfindungsgemäßen medizinischen Matratze;
- FIG 2: eine Ansicht einer Anordnungsmatrix für Luftkanäle einer medizinischen Matratze;
- FIG 3: ein Schnitt durch die Matratze aus FIG 2 von vorne;
- FIG 4: ein Schnitt durch die Matratze aus FIG 2 von der Seite;
- FIG 5: ein vergrößertes Schnittbild durch eine erfindungsgemäße medizinische Matratze;
- FIG 6: ein medizinisches Bildgebungsgerät mit einer medizinischen Matratze;
- FIG 7: eine Ansicht eines Positionsprofils, und
- FIG 8: eine Abfolge des erfindungsgemäßen Verfahrens.

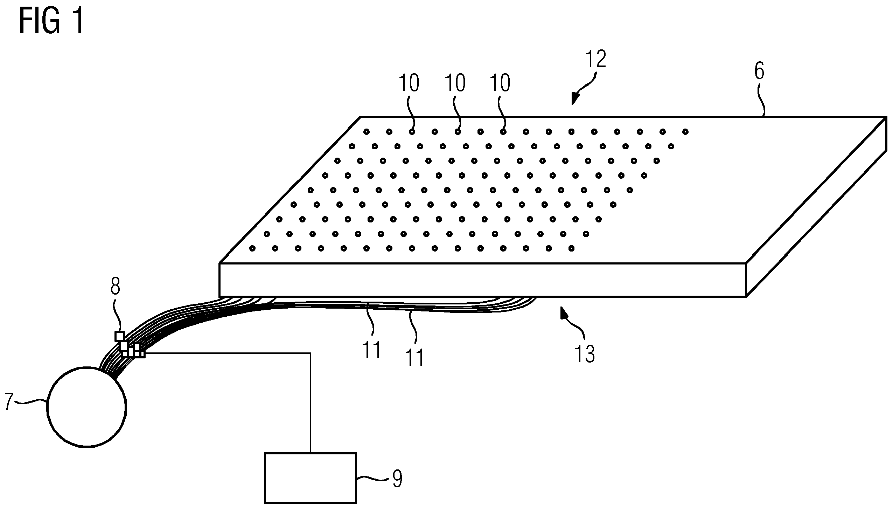

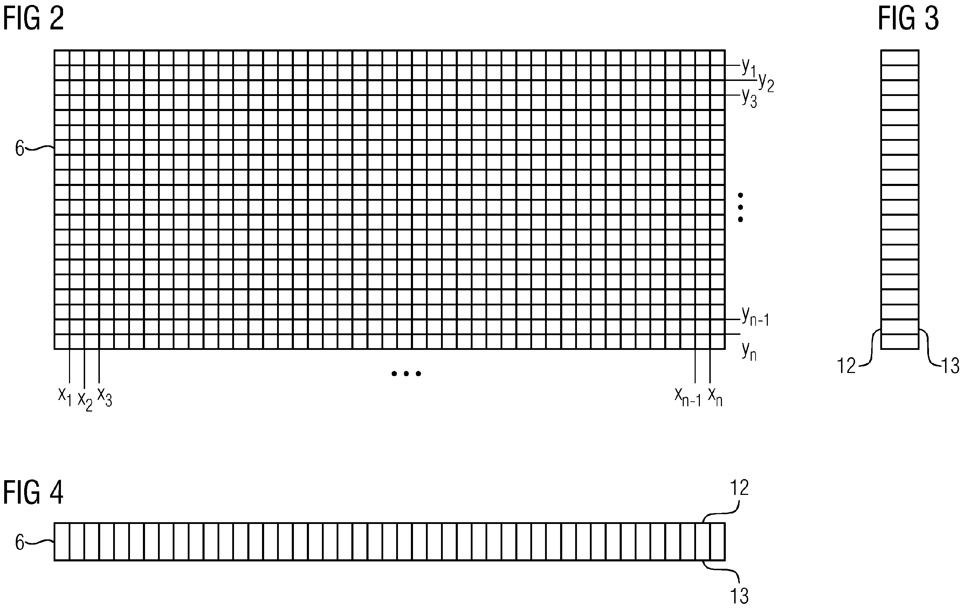

In der FIG 1 ist eine medizinische Matratze 6 mit einer Oberseite 12 zur Lagerung eines Patienten gezeigt. Die medizinische Matratze 6 weist eine Vielzahl von Luftkanälen 10 auf, welche sich durch die Matratze von einer Unterseite 13 zur Oberseite 12 der Matratze 6 erstrecken und - wie in FIG 2, 3 und 4 gezeigt - matrixartig über zumindest einen Teilbereich der Matratze erstrecken. Matrixartig bedeutet hierbei, dass an jedem Schnittpunkt der Achsen xᵢ und yᵢ, also z.B. bei den Schnittpunkten von x₁/y₁, X₂/y₁, X₃/y₁, ... xₙ/y₁ ... xₙ/yₙ, ein Luftkanal 10 angeordnet ist. Der Teilbereich, über den sich die Matrix erstreckt, nimmt dabei insbesondere mindestens 50% der Oberfläche der Oberseite der Matratze ein, kann sich auch über die gesamte Oberfläche erstrecken.

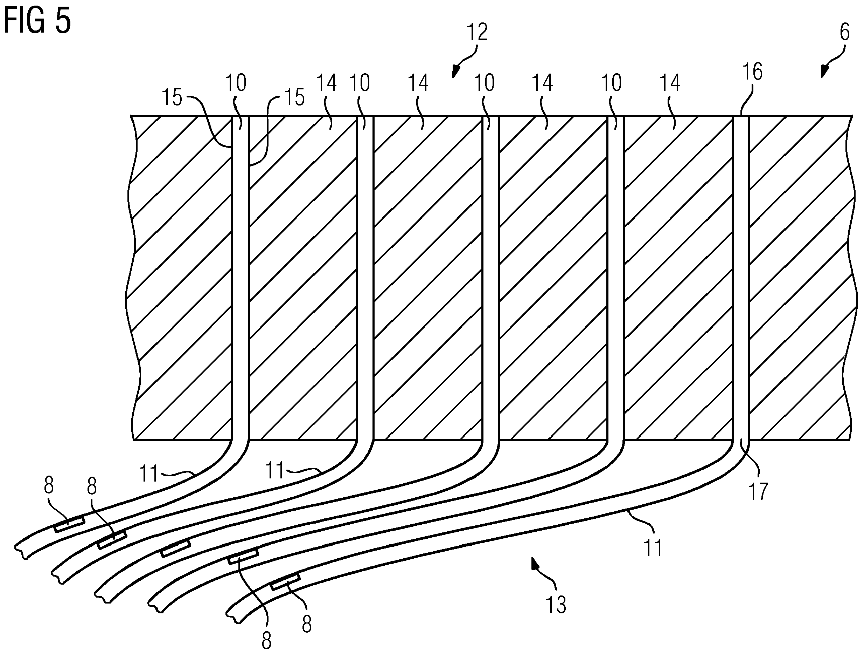

Jeder der Luftkanäle 10 weist an seinem ersten Ende 16 - wie in der vergrößerten Ansicht in FIG 5 gezeigt - im Bereich der Oberseite eine Öffnung auf und ist an seinem zweiten Ende 17 an einen Luftversorgungsschlauch 11 angeschlossen. Die Wandungen 15 der Luftkanäle 10 sind luftdicht abgeschlossen aufgebaut, so dass Luft, die durch den Luftversorgungsschlauch 11 in den Luftkanal gepumpt wird, lediglich durch die Öffnung im Bereich der Oberseite entweichen kann und im Falle eines teilweisen oder kompletten Verschlusses durch einen Patienten einen Druck erzeugt. Die Luftkanäle 10 sind außerdem komprimierbar ausgebildet. Mittels einer Luftversorgungseinrichtung 7, zum Beispiel eines Kompressors oder einer Pumpe, wird Luft durch die Luftversorgungsschläuche in die Luftkanäle 10 gedrückt. Außerdem sind Sensoren 8 zur Messung von Strömungs- und/oder Strömungsänderungskenngrößen der Luftkanäle 10 vorgesehen. Die Sensoren 8 messen entweder kontinuierlich oder in festgelegten zeitlichen Abständen die jeweiligen Kenngrößen.

Die Luftkanäle 10 sind außerdem röntgentransparent ausgebildet, ebenso die Luftversorgungsschläuche 11. Das Material, aus dem die Matratze zwischen den Luftkanälen 10 ausgebildet ist, ist luftdurchlässig ausgebildet, um eine Kompression der Matratze möglich zu machen. Das Material kann z.B. ein Schaumstoff 14 sein, z.B. ein elastomerer Schaum (Polyurethan-Weichschaum, Acrylnitril-Butadien-Kautschuk) oder ein thermoplastischer Schaum (PS-E, PP-E, PVC-E), welcher ebenfalls röntgentransparent ist. Die Matratze ist insbesondere im möglichen Röntgenstrahl-Durchleuchtungsbereich röntgentransparent ausgebildet. Die Sensoren 8 sind, sofern sie nicht auch röntgentransparent sind, derart angeordnet, dass sie außerhalb des Röntgenstrahl-Durchleuchtungsbereichs angeordnet sind oder angeordnet werden können. Wenn die Sensoren z.B. am Ende der Luftversorgungsschläuche angebracht sind, können die Luftversorgungsschläuche gebündelt werden. Dann können die Sensoren seitlich der Matratze (rechts oder links der Matratze oder auf beiden Seiten) oder im Fußbereich eines Patiententisches, auf welchem die medizinische Matratze aufliegt, angeordnet werden, so dass sie sich während einer Röntgenuntersuchung unterhalb des Röntgendetektors befinden.

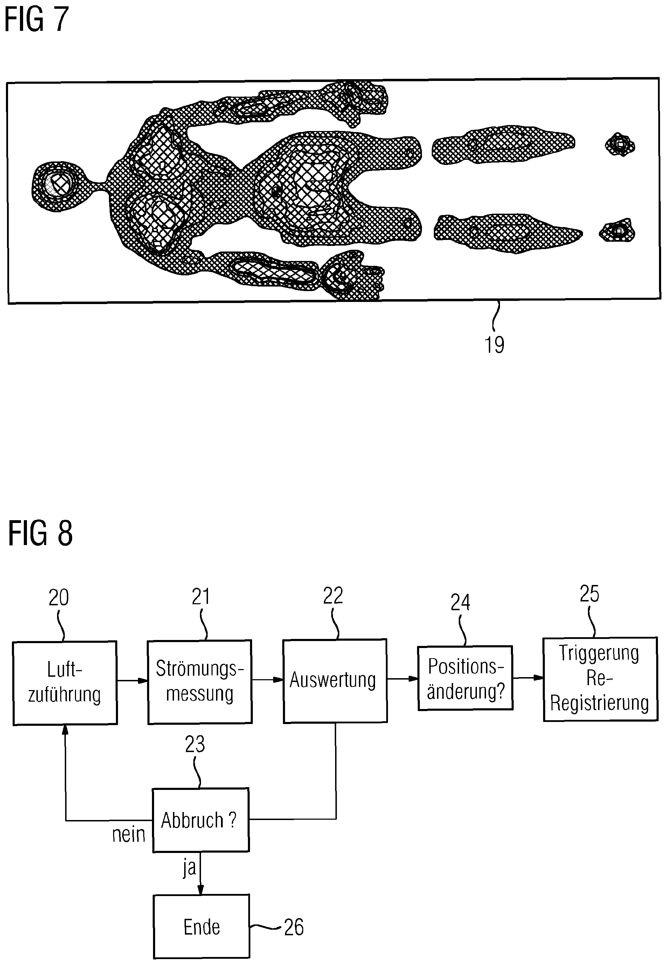

Durch unterschiedliche Matratzenbelastung seitens eines auf der Matratze angeordneten Patienten an verschiedenen Stellen der Matratze wird die luftdurchlässige Struktur mehr oder weniger stark komprimiert was zu einem der Belastung entsprechenden Druckabfall über dem jeweiligen Luftkanal führt. Aus den Kenngrößen kann dann durch eine Auswerteeinheit 9 und z.B. mittels Software oder einem Algorithmus ein Positionsprofil des Patienten oder Positionsänderungen bzw. Bewegungen des Patienten ermittelt werden. Hierzu können z.B. auch Machine Learning oder Deep Learning Algorithmen verwendet werden, welche bereits mit einer Vielzahl von Datensätzen von Probanden trainiert wurden, um aus den Kenngrößen-Mustern des Patienten die tatsächliche Position oder Bewegung des Patienten zu identifizieren. Bewegungen können dann z.B. als Atem- oder Herzbewegungen oder sonstige Bewegungen interpretiert werden. Das Ergebnis der Auswertung der von den Sensoren gelieferten Strömungs- und/oder Strömungsänderungskenngrößen kann z.B. ein Positionsprofil 19 wie in FIG 7 gezeigt sein. Die Genauigkeit ist dabei abhängig von der Dimension der Matrix von Luftkanälen bzw. der Granularität der Kammern der Matratze zwischen den Luftkanälen.

Durch die medizinische Matratze 6 kann ohne zusätzliche Kameratechnik eine effektive Echtzeit-Überwachung der Position des Patienten durchgeführt werden. Es können sowohl die initiale Position des Patienten als auch in Echtzeit Veränderungen bzw. Bewegungen oder Verschiebungen detektiert werden. Bei Verwendung der Matratze während Röntgenuntersuchungen, Therapien und interventionellen Eingriffen ist so eine optimale Patientenversorgung möglich.

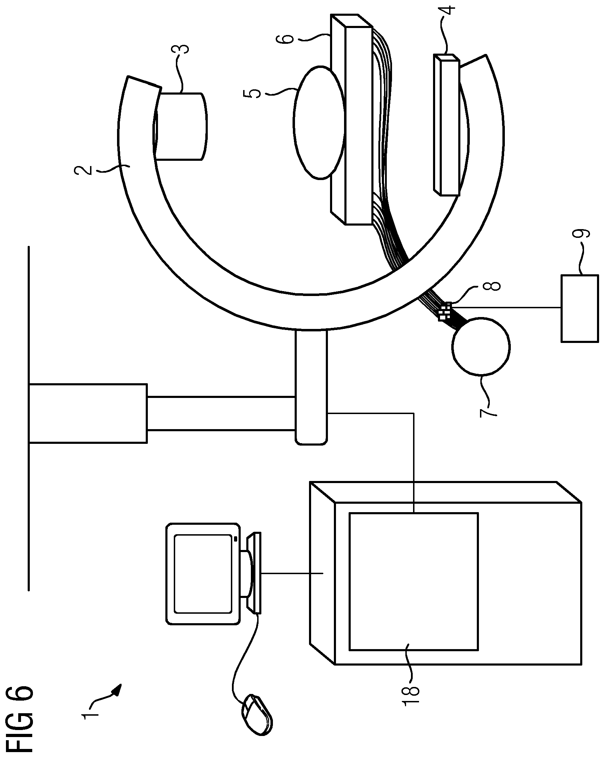

In der FIG 6 ist ein medizinisches Bildgebungsgerät 1 gezeigt, welches den auf der Matratze angeordneten Patienten 5 durchleuchtet. Das medizinische Bildgebungsgerät 1 weist z.B. einen bewegbaren C-Bogen 2 mit einer Röntgenquelle 3 und einem Röntgendetektor 4 auf und wird von einer Systemsteuerung 18 angesteuert. Die Sensoren der Matratze und die Luftversorgungseinrichtung 7 sind dabei seitlich der Matratze außerhalb des Röntgenstrahl-Durchleuchtungsbereichs der Röntgenquelle 3 angeordnet. Nicht gezeigt ist ein Patiententisch, auf welchem die Matratze und der Patient gelagert sind. Die Sensoren der Matratze und die Luftversorgungseinrichtung 7 können auch in einem Fußbereich des Patiententischs angeordnet sein. Die Auswerteeinheit 9 der Matratze kann auch mit der Matratze lediglich in Kommunikationsverbindung stehen und die Messdaten bzw. Kenngrößen von den Sensoren übermittelt bekommen. Die Auswerteinheit kann z.B. auch Teil des medizinischen Bildgebungsgeräts 1 sein.

In der FIG 8 ist ein Ablauf des erfindungsgemäßen Verfahrens zur Echtzeitüberwachung einer Positionierung eines Patienten während einer medizinischen Diagnose und/oder Therapie mit einem medizinischen Bildgebungsgerät unter Verwendung der beschriebenen Matratze, auf welcher der Patient gelagert ist, gezeigt. In einem ersten Schritt 20 wird mittels der Luftversorgungseinrichtung Luft über die Luftversorgungsschläuche in die Luftkanäle gepumpt. Durch einen auf der Oberseite der Matratze gelagerten Patienten wird diese entsprechend dem Gewicht des Patienten eingedrückt und die Luftkanäle werden entsprechend miteingedrückt und viele Luftkanäle werden teilweise oder vollständig verschlossen. Dadurch ändern sich die Strömungsverhältnisse in den jeweiligen Luftkanälen im Vergleich zu nicht-eingedrückten und nicht-verschlossenen Luftkanälen. In einem zweiten Schritt 21 werden in den Luftkanälen Strömungskenngrößen und/oder Strömungsänderungskenngrößen gemessen. Diese werden dann in einem dritten Schritt 22 von der Auswerteeinheit 9 ausgewertet, und zwar hinsichtlich einer Position und/oder Positionsänderung des Patienten.

Es kann aus den Kenngrößen z.B. ein Positionsprofil des Patienten erstellt werden, z.B. unter Verwendung von Software und/oder einem vortrainierten Machine Learning Algorithmus. Das so erstellte Positionsprofil kann z.B. für eine Registrierung der Patientenposition mit dem Bildgebungsgerät bzw. mit zuvor aufgenommenen Volumenbildern des Patienten verwendet werden. Sobald ein Abbruchkriterium 23 zutrifft (z.B. eine zuvor eingestellte Zeit oder eine Usereingabe o.ä.), kommt das Verfahren zu seinem Ende 26. Trifft kein Abbruchkriterium 23 zu, so wird das Verfahren weitergeführt. Während die Luftzuführung insbesondere kontinuierlich stattfindet, können die Messung des zweiten Schrittes 21 und die Auswertung des dritten Schrittes 22 kontinuierlich oder in vorgegebenen zeitlichen Abständen durchgeführt werden, je nach Bedarf oder Voreinstellung.

Aus den Kenngrößen können auch Positionsänderungen ausgewertet werden, wenn der Patient z.B. verrutscht oder durch zyklische oder spontane Bewegungen seine Position ändert. Hier können Positionsänderungen einzeln ausgewertet werden oder es wird ein neues Positionsprofil erstellt und mit dem bisherigen verglichen, um Positionsänderungen abzuleiten. All dies kann bei Bedarf, also z.B. bei einer fluoroskopischen Untersuchung, im Rahmen einer Operation oder eines interventionellen Eingriffes, live und in Echtzeit durchgeführt werden. Sobald ein Abbruchkriterium 23 zutrifft (z.B. eine zuvor eingestellte Zeit oder eine Usereingabe o.ä.), kommt das Verfahren zu seinem Ende 26.

Außerdem kann, sobald in einem optionalen vierten Schritt 24 eine Positionsänderung abgefragt und detektiert wird, ein Hinweis oder eine Warnung weitergegeben werden. Es kann auch alternativ bei detektierter Positionsänderung in einem optionalen fünften Schritt 25 eine Re-Registrierung des medizinischen Bildgebungsgeräts mit der Patientenposition getriggert werden.

Die erfindungsgemäße Matratze weist eine Vielzahl von Vorteilen gegenüber dem Stand der Technik auf: Es kann eine initiale Registrierung der Position des Patienten aufgrund von Information der Matratze (und der Systemposition des Bildgebungsgeräts) mit vorliegenden 3D-Volumenbildern automatisch durchgeführt werden. Es kann ein Live Monitoring von Patientenbewegungen erfolgen und als Warnung für medizinisches Personal oder das Bildgebungsgerät verwendet werden. Infolgedessen kann eine bestehende Registrierung automatisch korrigiert werden. Regelmäßige Druckschwankungen im Thorax- und Abdomenbereich des Patienten können als Atemtriggerung ausgewertet werden. Weitere Überwachungsgeräte im Interventionsraum sind nicht notwendig bzw. können eingespart werden (wie z.B. Kameras). Die Matratze weist keine bildstörende Elektronik im Röntgenstrahl-Durchleuchtungsbereich auf, so dass die Bildgebung unbeeinflusst hohe Qualität aufweist. Optional kann durch die Matratze eine temperaturgeregelte Belüftung zum Wärmen des Patienten während der Untersuchung durchgeführt werden. Außerdem ist eine Ableitung des Patientengewichts und der Größe aus (kalibrierten) Daten möglich.

Die Erfindung lässt sich in folgender Weise kurz zusammenfassen: Medizinische Matratze zur Bestimmung von Position und/oder Positionsänderung eines auf der Matratze gelagerten Patienten, welche Matratze zumindest teilweise aus röntgendurchlässigen Materialien ausgebildet ist, aufweisend eine Oberseite, welche als Liegefläche für den Patienten ausgebildet ist und eine der Oberseite gegenüber liegende Unterseite, eine Vielzahl von Luftkanälen, welche über zumindest einen Teilabschnitt der Matratze in einer Matrix verteilt derart angeordnet ist, dass die Luftkanäle sich von der Unterseite zur Oberseite der Matratze erstrecken, wobei jeder Luftkanal an seinem ersten Ende im Bereich der Oberseite eine Öffnung aufweisen und an seinem zweiten Ende an einen Luftversorgungsschlauch angeschlossen ist, eine Luftversorgungseinrichtung, welche über die Luftversorgungsschläuche Luft in die Luftkanäle pumpt, Sensoren zur Messung von Strömungs- und/oder Strömungsänderungskenngrößen in den Luftkanälen, und eine zugeordnete Auswertungseinheit zur Auswertung der von den Sensoren gemessenen Strömungskenngrößen und/oder Strömungsänderungskenngrößen hinsichtlich einer Position und/oder Positionsänderung des auf der Matratze gelagerten Patienten

## Patentansprüche

1. Medizinische Matratze (6) zur Bestimmung von Position und/oder Positionsänderung eines auf der Matratze (6) gelagerten Patienten (5), welche Matratze (6) zumindest teilweise aus röntgendurchlässigen Materialien ausgebildet ist, aufweisend
• eine Oberseite (12), welche als Liegefläche für den Patienten (5) ausgebildet ist und eine der Oberseite (12) gegenüber liegende Unterseite (13),
• eine Vielzahl von Luftkanälen (10), welche über zumindest einen Teilabschnitt der Matratze (6) in einer Matrix verteilt derart angeordnet ist, dass die Luftkanäle (10) sich von der Unterseite (13) zur Oberseite (12) der Matratze (5) erstrecken, wobei jeder Luftkanal (10) an seinem ersten Ende (16) im Bereich der Oberseite (12) eine Öffnung aufweisen und an seinem zweiten Ende (17) an einen Luftversorgungsschlauch (11) angeschlossen ist,
• eine Luftversorgungseinrichtung (7), welche über die Luftversorgungsschläuche (11) Luft in die Luftkanäle (10) pumpt,
• Sensoren (8) zur Messung von Strömungskenngrößen und/oder Strömungsänderungskenngrößen in den Luftkanälen (10), und
• eine zugeordnete Auswertungseinheit (9) zur Auswertung der von den Sensoren (8) gemessenen Strömungskenngrößen und/oder Strömungsänderungskenngrößen hinsichtlich einer Position und/oder Positionsänderung des auf der Matratze (6) gelagerten Patienten (5).

2. Medizinische Matratze nach Anspruch 1, wobei die Sensoren (8) von Strömungssensoren oder Drucksensoren gebildet werden.

3. Medizinische Matratze nach einem der vorangehenden Ansprüche, wobei die Auswertungseinheit (9) zur Erstellung eines Positionsprofils des Patienten (5) ausgebildet ist, insbesondere unter Verwendung von Machine Learning Methoden.

4. Medizinische Matratze nach einem der vorangehenden Ansprüche, wobei die Auswertungseinheit (9) zur Echtzeit-Überwachung von Positionsänderungen des Patienten (5) ausgebildet ist, insbesondere unter Verwendung von Machine Learning Methoden.

5. Medizinische Matratze nach einem der vorangehenden Ansprüche, aufweisend eine Speichereinheit zur Speicherung von Daten und Messwerten.

6. Medizinische Matratze nach einem der vorangehenden Ansprüche, wobei die Luftversorgungseinrichtung (7) von einer Pumpe oder einem Kompressor gebildet wird.

7. Medizinische Matratze nach einem der vorangehenden Ansprüche, wobei die Sensoren (8) zur Messung von Strömungs- und/oder Strömungsänderungskenngrößen an oder in den Luftversorgungsschläuchen (11) angeordnet sind.

8. Medizinische Matratze nach Anspruch 7, wobei die Sensoren (8) außerhalb eines den Patienten (5) bestrahlenden Röntgenstrahls anordbar sind.

9. Medizinische Matratze nach Anspruch 7 oder 8, wobei die Luftversorgungsschläuche (11) außerhalb eines Röntgenstrahl-Durchleuchtungsbereichs, insbesondere seitlich der Matratze (6), zusammenführbar angeordnet sind.

10. Medizinische Matratze nach einem der vorangehenden Ansprüche, wobei die Matrix von Luftkanälen (10) sich über zumindest 50% der Matratze erstreckt.

11. Medizinische Matratze nach einem der vorangehenden Ansprüche, wobei die Matratze (6) zwischen den Luftkanälen (10) aus einem luftdurchlässigen Material, insbesondere Schaumstoff, gebildet ist.

12. Medizinische Matratze nach einem der vorangehenden Ansprüche, wobei die Wandungen (15) der Luftkanäle (10) röntgentransparent und komprimierbar ausgebildet sind.

13. Verfahren zur Echtzeitüberwachung einer Positionierung eines Patienten (5) während einer medizinischen Diagnose und/oder Therapie mit einem medizinischen Bildgebungsgerät (1) unter Verwendung einer Matratze (6) nach einem der Ansprüche 1 bis 12, auf welcher der Patient gelagert ist, mit den folgenden Schritten:
• Einpumpen von Luft in die Luftkanäle (10) durch die Luftversorgungseinrichtung (7) über die Luftversorgungsschläuche (11),
• Messung von Strömungs- und/oder Strömungsänderungskenngrößen in den Luftkanälen (10), und
• Auswertung der von den Sensoren (8) gemessenen Strömungs- und/oder Strömungsänderungskenngrößen hinsichtlich einer Position und/oder Positionsänderung des Patienten (5),
wobei die Schritte bis zu einem Abbruchkriterium kontinuierlich und/oder in vorgegebenen zeitlichen Abständen durchgeführt werden.

14. Verfahren nach Anspruch 13, wobei aus den gemessenen Strömungs- und/oder Strömungsänderungskenngrößen ein Positionsprofil erstellt wird und für eine Registrierung des medizinischen Bildgebungsgeräts (1) mit der Patientenposition verwendet wird.

15. Verfahren nach Anspruch 13 oder 14, wobei ein Hinweis oder eine Warnung weitergegeben wird, wenn die Auswertung eine Positionsänderung des Patienten (5) ergibt.

16. Verfahren nach einem der Ansprüche 13 bis 15, wobei eine Re-Registrierung des medizinischen Bildgebungsgeräts (1) mit der Patientenposition getriggert wird, wenn die Auswertung eine Positionsänderung des Patienten (5) ergibt.
